# EUROPEAN PATENT SPECIFICATION

(11) **EP 0 393 640 B1**
(45) Date of publication and mention of the grant of the patent: **30.08.1995**
(21) Application number: 90107374.2
(22) Date of filing: 18.04.1990
(51) Int. Cl.: C12P 21/08, G01N 33/577, G01N 33/68, C12N 5/16

(54) **A monoclonal antibody recognizing C-terminus of ANP**
Monoklonale Antikörper gegen den C-Terminus von ANP
Anticorps monoclonaux reconnaissant l'extrémité C-terminale de l'ANP

(30) Priority: 19.04.1989 JP 99620/89
(43) Date of publication of application: 24.10.1990
(73) Proprietor: SHIONOGI SEIYAKU KABUSHIKI KAISHA trading under the name of SHIONOGI & CO. LTD., Osaka 541 (JP)
(72) Inventor: Misaki, Atsushi, Osaka-shi, Osaka (JP); Yamauchi, Akira, Mino-shi, Osaka (JP); Kono, Masao, Osaka (JP); Igano, Ken'ichi, Nara (JP); Inouye, Ken, Kobe-shi, Hyogo (JP)
(74) Representative: VOSSIUS & PARTNER

(56) References cited:
- EP-A- 0 306 309
- EP-A- 0 351 244

## Description

The present invention relates to a monoclonal antibody recognizing the C-terminus of ANP, hybridomas producing the monoclonal antibody and an enzyme immunoassay (abbreviated as EIA, hereinafter) and radioimmunoassay (abbreviated as RIA, hereinafter) of ANP characterized by sandwiching ANP with a monoclonal antibody recognizing the C-terminus of ANP and an antibody recognizing the N-terminus of ANP.

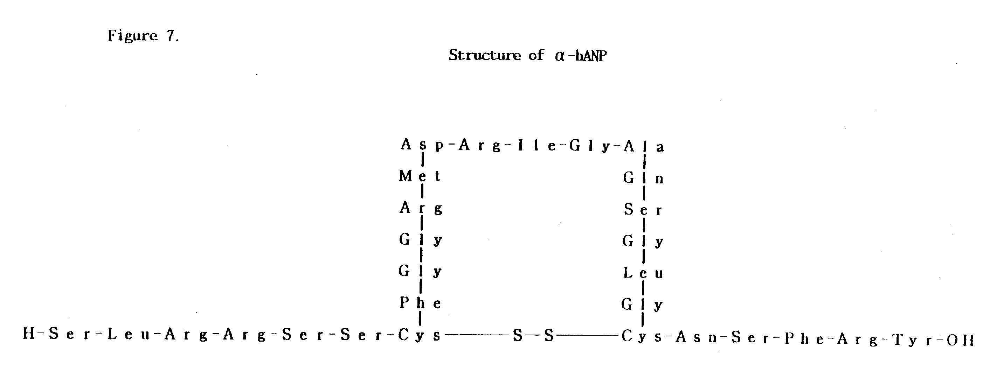

An atrial natriuretic polypeptide (ANP) is produced by cells of atrial myocyte, is included in granules and has strong diuretic and natriuretic actions. The polypeptides like this are found not only in humans but also in rats and they are named hANP and rANP, respectively. Further, hANP and rANP can be devided into 3 groups, namely, α-, β-, and γ-type, respectively. α-hANP consists of 28 amino acid residues and its Cys[7] and Cys[23]-(cysteine residues at 7th and 23rd positions from N-terminus, respectively) form disulfide bond and a sequence between them form a ring structure (Biochem. Biophys. Res. Commun. (abbreviated as BBRC hereinafter) 118, 131-139, 1984) (See Fig 7). A 12th residue from N-terminal of α-hANP is Met, whereas a 12th amino acid residue from N-terminus is Ile in α-rANP (BBRC 117, 839-865, 1983). β-hANP is an antiparallel dimer of α-hANP (JP Unexamd. Pat. Publn. No. 60-184098). γ-hANP consists of 126 amino acid residues and its amino acid sequence between 66th and 126th at the C-terminus corresponds to α-hANP.

As regards an assay of ANP, a radioimmunoassay using an antiserum has already been established (Science 228, 323-325, 1985; Nature 314, 264-266, 1985; BBRC 124, 815-821, 1984; BBRC 124, 663-668, 1984; BBRC 125, 315-323, 1984).

In known antisera, it is known that antiserum CR-3 recognizes a C-terminal fragment [17-28] of ANP. Further, monoclonal antibodies having different kinds of specificity for ANP have recently been reported. (Life Sci. 38, 1991-1997, 1986; Mol. Immunol. 24, 127-132, 1987; Endocrinology 121, 843-852, 1987; Hybridoma 6, 433-440, 1987). Especially, KY-ANP-I recognizing N-terminus site [7-16] of ANP (EP-A-0 306 309) is known as monoclonal antibody recognizing N-terminus of ANP. Furthermore, a method for immunoassay of α-ANP using the KY-ANP-I and an antiserum recognizing C-terminus has already been established (EP-A-0 306 309). EP-A-0 351 244 discloses antibodies that recognize the C-terminal side in the cyclic region of α-ANP. However, a monoclonal antibody recognizing the C-terminus of α-ANP and β-ANP has not been obtained yet.

In a conventional immunoassay of ANP it has been necessary to extract ANP from a test sample, and therefore, a monoclonal antibody having a high affinity and enabling an exact assay without extraction is demanded. Since the KY-ANP-I described above is known to recognize N-terminus of ANP, when a monoclonal antibody recognizing the C-terminus of α-ANP is available, a sandwich immunoassay using two types of monoclonal antibody recognizing N- and C-termini of α-ANP, respectively, is realized.

Antiserum CR-3 has already been known as specifically recognizing the C-terminus. However, the antiserum is polyclonal and, therefore, inferior to monoclonal antibodies in terms of stable supply, uniform specificity and so on.

This invention provides a monoclonal antibody recognizing a sequence of the C-terminus of α-ANP or β-ANP, that is, a portion included in a fragment of α-ANP [17-28] and hybridomas producing the monoclonal antibody. Furthermore, the present invention relates to an enzyme immunoassay (EIA) and radioimmunoassay (RIA) of ANP characterized by sandwiching ANP with a monoclonal antibody recognizing C-terminus of ANP and an antibody recognizing the N-terminus of ANP.

This monoclonal antibody specifically recognizing the C-terminus ANP and having high affinity for it is useful for sandwich immunoassay of ANP.

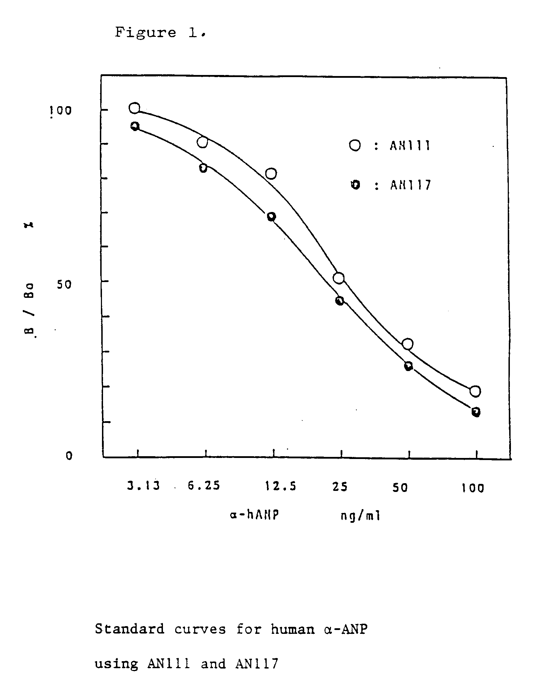

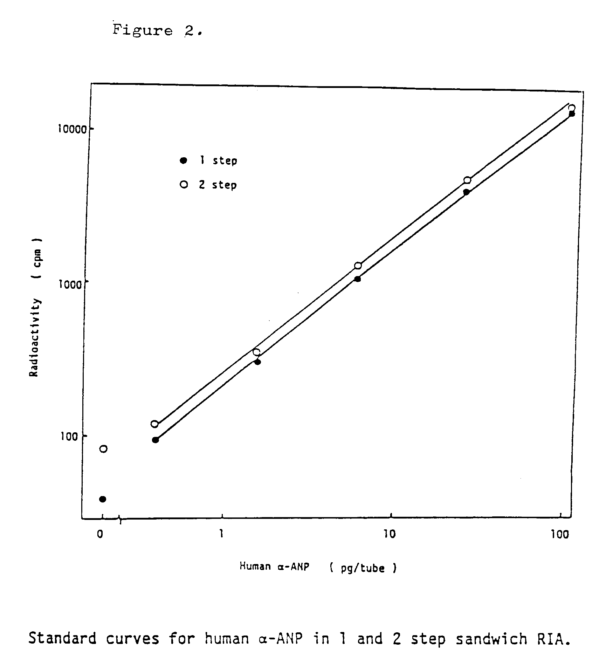

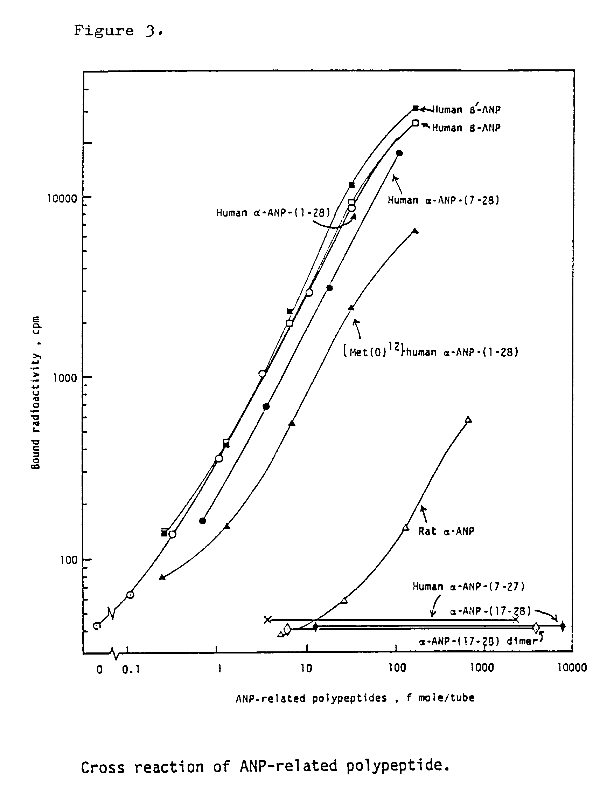

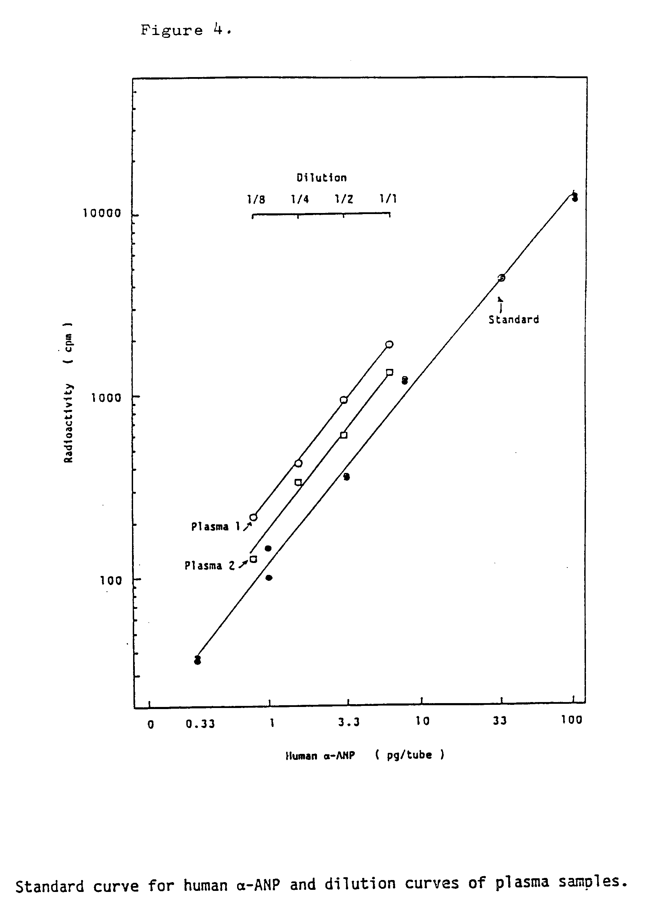

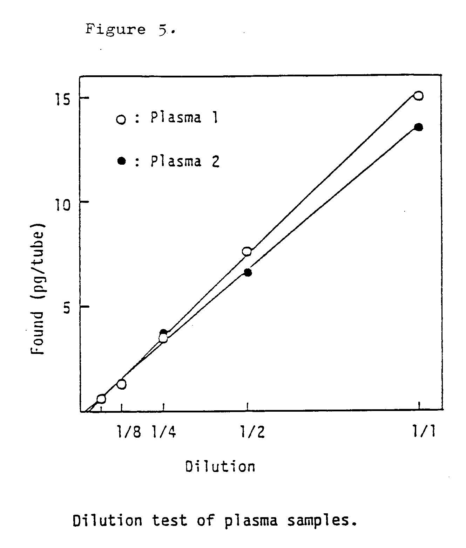

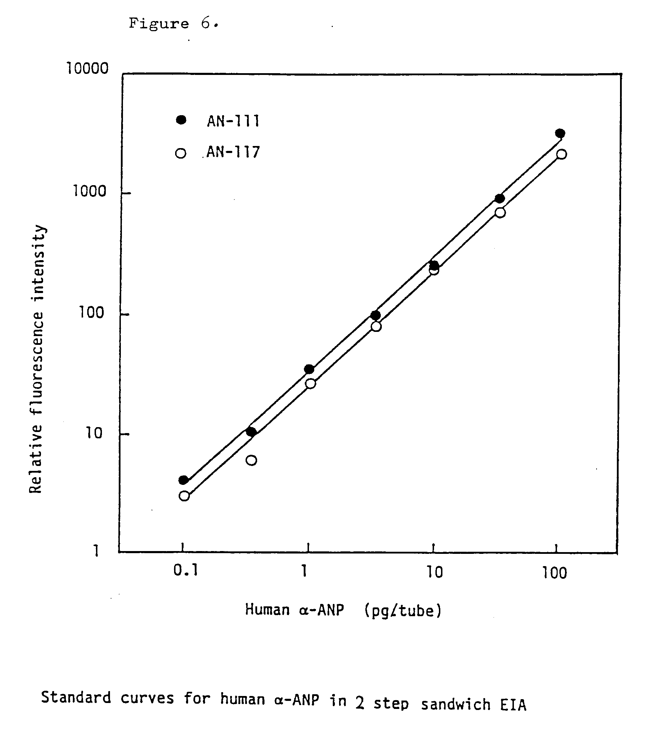

Figure 1 displays standard curves of α-hANP using AN111 and AN117. Figure 2 shows standard curves of α-hANP in sandwich radioimmunoassays of one-step and two-step. Figure 3 denotes cross-reactivity of ANP-related peptides. Figure 4 indicates a standard curve and plasma dilution curve of α-hANP. Figure 5 illustrates the result of the dilution test of plasma. Figure 6 shows a standard curve of α-hANP in sandwich enzyme immunoassay (two-step). Figure 7 exhibits structure of α-hANP.

The technical problem underlying the present invention is to provide a monoclonal antibody having a high affinity for the C-terminus of α-ANP and β-ANP. The solution of the above technical problem is achieved by providing monoclonal antibodies recognizing the C-terminus of ANP which are obtainable from the hybridomas AN111 (FERM BP-2197) or AN117 (FERM BP-2198). This monoclonal antibody can then be used in diagnostic assays or methods to detect ANP in test samples, e.g. together with a monoclonal antibody that recognizes the N-terminus of ANP to provide a highly sensitive sandwich immunoassay to detect the presence of ANP in a test sample without first extracting the ANP.

### (1) Preparation of Hybridoma Producing Monoclonal Antibody

Alpha-hANP consists of 28 amino acid residues and shows low antigenicity because of its lower molecular weight. Accordingly, it is conjugated with bovine serum albumin, bovine thyroglobulin, and the like to be used as an immunogen. The resulting conjugate is emulsified in an appropriate adjuvant for the immunization of mice.

The above emulsion is peritoneally, subcutaneously or intraveneously inoculated into mice repeatedly at an interval of a few weeks for the immunization of mice. The spleen is taken 3 to 5 days after the last immunization to be used as antibody-producing cells.

Myeloma cells having a suitable marker such as hypoxanthine - guanine - phosphoribosyl transferase deficiency (HGPRT⁻), thymidine kinase deficiency (TK⁻), etc. are used as parent cells which are fused with antibody-producing cells to prepare hybridomas.

By the fusion of the antibody-producing cells and the myeloma cells, hybridomas are prepared.

Media for the preparation of hybridomas include Eagle's MEM, Dulbecco's modified medium, RPMI-1640, and usually used media, if necessary, fortified with approximately 10 - 30 % fetal calf serum (FCS).

Myeloma cells and spleen cells as parent cells are prepared at a ratio of ca. 1:5. As a fusing agent, polyethylene glycol is used at a concentration of 50 % for the efficient fusion. Resulting cells are selected by the HAT method. Screening of hybridomas so obtained is performed according to membrane fluorescent antibody technique, Enzyme Linked Immunosorbent Assay, immunohistochemistry technique or other usual assays to select clones of hybridomas producing desired immunoglobulin. In order to ensure the unity of hybridomas, the hybridomas are incubated in 96-well microplate with normal spleen cells as a feeder layer at a concentration of less than one hybridoma per well. On the clones formed, the same screening as described above is performed again. This subcloning process is repeated to obtain a uniform hybridoma.

### (2) Production of Monoclonal Antibody

In order to produce the monoclonal antibody of this invention, the hybridoma as obtained above is cultured in vitro or in vivo. In the case of in vitro culture, the hybridoma is cultured in an ordinary medium supplemented with FCS as mentioned above for 3 - 5 days to recover a monoclonal antibody from the supernatant. In the case of in vivo culture, the hybridoma is implanted to the abdominal cavity of a mammal, 7 - 14 days after which the ascitic fluid is collected to recover a monoclonal antibody therefrom. In the case of the in vivo culture, a much larger quantity of the monoclonal antibody can efficiently be obtained than that in the case of the in vitro culture and the in vivo culture is therefore preferable.

The monoclonal antibody so obtained from the supernatant or ascitic fluids can be purified by sodium sulfate-fractionation, DEAE-sepharose column chromatography, other known methods or their combinations, for instance, by the method to be described in the following Examples.

As clear from the following Examples, the monoclonal antibodies AN111 and AN117 of this invention each recognizes α-ANP and β-ANP. The epitope seems to be the C-terminus of α-ANP, specifically, a portion between Ala (17) and Tyr (28) of α-ANP. Since the portion from Ala (17) to Tyr (28) is common to γ-ANP, the monoclonal antibody of this invention must naturally recognize γ-ANP. In conclusion, the monoclonal antibody is AN111 and AN117 of this invention recognize all the types of ANP, that is, α-, β-, and γ-ANPs.

The monoclonal antibodies of this invention scarcely react with the followings as apparent from the Table 1.
α-hANP[7 - 27],
α-hANP[17 - 22],
α-hANP[17 - 20],
α-hANP[21 - 26], dimer, and
α-rANP[7 - 23].

The disappearance of the reactivity seems due to the deficiency of the followings.
α-ANP[28]
α-ANP[23 - 28],
α-ANP[21 - 28],
α-ANP[27 - 28], and
α-ANP[24 - 28].

Accordingly, the monoclonal antibodies of this invention are deduced to recognize a portion contained in the above fragments. Taking such a fact into the consideration that α-ANP (17-28) is used as an immunogen, the monoclonal antibodies of this invention are concluded to recognize a portion contained in the following fragment:
α-ANP[ n - 28]
wherein n is an integer from 17 to 28.
The integer n is preferably 17, 21, 23, 24, 27, or 28.

In this specification, for example, α-hANP[17 - 28] means the fragment from 17th Ala to 28th Tyr of α-hANP.

The monoclonal antibodies of this invention display high affinities with α-ANP; AN111 and AN117 display affinity constants of 1.7 × 10⁹ M⁻¹ and 2.3 × 10⁹ M⁻¹, respectively. Calculated from the standard curves of α-ANP in Figure 1, 90 % inhibition concentrations (IC₉₀) of AN111 and AN117 are 6.2 ng/ml and 4.4 ng/ml, respectively.

The hybridoma AN111 and AN117 producing the monoclonal antibodies AN111 and AN117 of this invention have been deposited under accession numbers FERM BP-2197 and FERM BP-2198, respectively, with Fermentation Research Institute at 1-3, Higashi 1-chome, Tsukuba-shi, Ibaraki, 305 Japan since December 20, 1988 according to the Budapest Treaty.

### (3) Immobilization of Antibody on Solid Phase

As a solid phase on which an antibody is immobilized, a commercially available ordinary carrier for the antigen-antibody reaction such as bead, ball, tube and plate of glass or synthesized resin can be used. On these carriers, an antibody recognizing N-terminus or C-terminus of α-hANP is immobilized by allowing them to stand overnight in phosphate buffer etc. of pH 6 - 10, preferably around neutral, at room temperature. The carrier adsorbing the antibody is stored with cooling in the presence of preservatives such as sodium azide.

Any of monoclonal and polyclonal antibodies can be immobilized on a carrier by the above method.

### (4) Preparation of Enzyme-Conjugated Antibody

### Preparation of Fab' Fragment

F(ab')₂ fragments obtained by the pepsin-digestion of IgG are reduced with 2-mercaptoethanol to prepare desired Fab' fragments. Preparation of Fab' fragment from IgG is detailed in J. Immunoassay, 4, 209 - 327 (1983), which is applicable to this invention.

### Conjugate of Enzyme to Antibody

As an enzyme to be conjugated to an antibody, alkaline phosphatase, β-D-galactosidase, peroxidase, glucose-oxidase is preferable and, especially, horseradish peroxidase is more preferable. As a bridging agent, N,N'-O-phenylene dimaleimide, N-succinimidyl 4-(N-maleimidomethyl)cyclohexane-1-carboxylate, N-succinimidyl 6-maleimidohexane-1-carboxylate, N-succinimidyl 3-( 2-pyridyldithio)propionate and other known bridging agents are applicable. The reactions of these bridging agents with enzymes and antibodies are performed according to the known methods depending upon the property of each bridging agent. In this reaction, an antibody itself and its fragment such as Fab', Fab, F(ab')₂, are applicable. In this invention, as a bridging agent, N-succinimidyl 4-(N-maleimidoethyl)cyclohexane-1-carboxylate was most preferable. An enzyme-conjugated antibody can be prepared by the above method from any of monoclonal and polyclonal antibodies. The enzyme-conjugated antibody so obtained is preferably purified by an affinity chromatograpby, whereby a more sensitive immunoassay system is realized. The purified enzyme-conjugated antibody is stored in darkness in the presence of a stabilizer such as thymelosal or after lyophilization.

### (5) Preparation of Radioisotope-labelled Antibody

An antibody is labelled with radioisotope such as ¹²⁵I according to the known method such as the chloramine T method.

### (6) Assay of ANP

Combination of antibodies in assaying ANP with use of the agents for an immunoassay as prepared above is as follows. Where an antibody recognizing the N-terminus of ANP is immobilized, an antibody recognizing the C-terminus is labelled. On the contrary, where an antibody recognizing the C-terminus is immobilized, an antibody recognizing the N-terminus is labelled. In general, a monoclonal antibody (i.e. AN111 and AN117 of this invention), a larger quantity of which can stably be produced, is suitable for the immobilization because a rather large quantity of an antibody is necessary for the immobilization.

Of course, a polyclonal antibody from an antiserum can be used for the immobilization without difficulties. An antibody to be labelled may be either monoclonal or polyclonal if it recognizes a portion different from that recognized by an immobilized antibody. For example, when AN111 or AN117 of this invention is immobilized, KY-ANP-I can be used as a labelled antibody and, when KY-ANP-I is immobilized then AN111 or AN117 can be used as a labelled antibody. Of course, an antiserum recognizing the N-terminus of α-ANP is also applicable to the assay of this invention

By combination of the antibody of this invention with KY-ANP-I as described above, an assay of α-ANP and β-ANP is realized as shown in the following Examples. Namely, the antibody of this invention is suitable for a sandwich immunoassay using KY-ANP-I. Further, if an antibody recognizing the N-terminus of γ-ANP is used as the antibody recognizing the N-terminus of ANP, a specific assay of γ-ANP is possible.

### Example 1

### Preparation of a hybridoma producing anti-α-hANP monoclonal antibody and production of the antibody

### (1) Preparation of conjugate for immunization.

To a mixture of bovine thyroglobulin (64.4 mg / 2.4 ml) and α-hANP [17-28] (1.8 mg / 1.7 ml) was added a solution of 1-ethyl-3-(3-dimethyl aminopropyl)carbodiimide hydrochloride (136 mg / 3.4 ml) and the mixture was allowed to react with agitating at 0 °C for 2 hr. The resultant was dialyzed against distilled water at 4 °C for 2 days. After removing precipitates by a centrifugation, the resultant was lyophilized to give 39.7 mg of α-hANP [17-28] - bovine thyroglobulin conjugate. A ratio of conjugation of α-hANP [17-28] per bovine thyroglobulin molecule was 29 : 1.

### (2) Immunization

A physiological saline suspension of α-hANP [17-28] - bovine thyroglobulin conjugate (a concentration of conjugate; 1.82 mg/ml) was emulsified with Freund's complete adjuvant equivalent to the same volume of the above suspension. The emulsion (0.1 ml) was subcutaneously administered 4 times to the back of Balb/c mouse (female, 8 week ages) at an interval of 3 weeks. The amount of the conjugate per mouse per administration was 91 »g and the amount as a hapten of α-hANP [17-28] was 5 »g. Furthermore, 3 weeks after the 4th immunization, 0.1 ml of physiological saline suspension of 181 »g (hapten 10 »g) of α-hANP [17-28] - bovine thyroglobulin conjugate was intraperitoneally administered once a day for 2 straight days as booster immunization.

### (3) Fusion of Cells

Three days after the booster immunization, the spleen of the mouse was picked out and the cells in 0.17 M ammonium chloride were allowed to stand for 5 min. to destroy erythrocytes. The remaining cells were suspended in RPMI-1640 medium to give spleen lymphocytes to be used in the following fusion. Next, 2.4 × 10⁷ myeloma cells (NS-1) resistant to 8-azaguanine suspended in RPMI-1640 medium and 1.2 × 10⁸ spleen lymphocytes were mixed and then centrifuged. To the obtained cell precipitate was added 0.8 ml of 50 % polyethylene glycol (molecular weight 4,000; Merck) in 1 min. with stirring by a top of a pipet and then the mixture was stirred for 1.5 min. To the mixture were added 2 ml of RPMI-1640 medium in 2 min. and then 2 ml of that in 1 min. Then 18 ml of RPMI-1640 was added dropwise with gentle agitation. After centrifugation and then removing the supernatant fluid, the obtained cell precipitate was suspended in 100 ml of HAI medium (20 % FCS - RPMI-1640 medium supplemented with 1 × 10⁻⁴ M hypoxanthine, 4 × 10⁻⁷ M aminopterine, and 1.6 × 10⁻⁵ M thymidine) and poured into 10 plates of 96-well microplate (Costar) at 0.1 ml per well. A suspension of mouse spleen cells in HAT medium had previously been poured into the microplates (5 × 10⁴ cells/0.1 ml/well). A half volume of the HAT medium was substituted by new one once per 2 - 3 days. After about 10 days, a proliferation of hybridoma was observed. The wells in which hybridoma grew were 951 (99 %).

### (4) Selection of Hybridoma

On supernatants of the wells in which hybridoma grew, whether anti-α-hANP antibody was produced or not was examined according to radioimmunoassay. 50 »ℓ of the supernatant of the culture, 100 »ℓ of 2 % bovine gamma globulin dissolved in an assay buffer (described in Example 5) and 100 »ℓ of ¹²⁵I-labelled α-hANP (about 30,000 cpm/ml, Amersham) diluted with the above assay buffer were mixed, and the mixture was incubated for 2 hr. at room temperature. To the mixture was added a solution of 250 »ℓ of 25 % polyethylene glycol 6,000 with stirring. After centrifugation (3,000 rpm, 20 min.), the radioactivity of the obtained precipitate without the supernatant was measured with gamma counter (Aroka ARC-600 type). Nine wells having more than 1,000 cpm of radioactivity were regarded as antibody-positive and selected.

### (5) Cloning

The hybridoma selected in Example 1 (4) was immediately cloned by the limiting dilution. The hybridoma was cultured at a concentration of 1 cell/ 200 »ℓ/well in 96-well microplate. The supernatant fluid in which the hybridoma has proliferated was subjected to the radioimmunoassay. The hybridoma having a character of antibody-positive was expanded. After repeating this cloning procedure twice or three times, a hybridoma AN111 and AN117, both of which produce anti-α-hANP antibody, suitable for the sandwich immunoassay described below were established.

### (6) Preparation of ascitic fluid

The hybridoma so established was intraperitoneally implanted to mice to prepare ascitic fluid, of a high concentration of antibody. The hybridomas (about 1 × 10⁷ cells) suspended in RPMI-1640 were intraperitoneally implanted to mice (Balb/c, female, to which 0.5 ml of pristane had intraperitoneally been administered 10 days before the above implantation). After collecting the ascitic fluid between 1 - 3 weeks after the implantation, the ascitic fluid was centrifuged to remove cells therein. To the resulting supernatant was added 0.1 % of sodium azide and the mixture was preserved under freezing. Ascitic fluids of AN111A and AN117A containing monoclonal antibodies produced by hybridomas AN111 and AN117, respectively, were obtained by the above method.

### (7) Purification of monoclonal antibody

Using affigel protein A MAPS-II® kit (Bio-Rad), the antibodies were purified from ascitic fluids of AN111A and AN117A, respectively. Using 1.2 ml of gel, each ascitic fluid (1 ml) was purified according to the recommended procedure and then 4.3 mg of the antibody was obtained from the AN111A and 2.5 mg from the AN117A. In order to determine a purity of antibody, SDS - polyacrylamide gel electrophoresis was performed. After reducing the antibody fraction with 2-mercaptoethanol, the resultant was subjected to 12.5 % polyacrylamide-gel electrophoresis. As a result, 2 bands, namely, H-chain and L-chain, are observed at a locations of molecular weights of about 52,000 and about 28,000, respectively.

### Example 2

### Determination of Class and Subclass of Monoclonal Antibody

Class and subclass of immunoglobulin produced by the hybridoma was determined by the immunodiffusion method using antibodies specific for each class or subclass of mouse immunoglobulin (Serotec, mouse monoclonal antibody-typing kit). As a result, in both the case of ascitic fluid AN111A and AN117A a precipitating line was only observed with anti IgG₁ antibody. Therefore, it was confirmed that both the antibodies belong to IgG₁.

### Example 3

### Affinity of Monoclonal Antibody (Binding Constant)

A standard curve of α-hANP was prepared in the same way of Example 5. Based on Scatchard's method, radioactivity of precipitate/radioactivity of supernatant as an ordinate and a concentration of α-hANP bound to with the antibody as an abscissa were plotted. The binding constant of the antibody was calculated from the inclination of the obtained line. As a result, the binding constant of AN111 was 1.7 × 10⁹ M⁻¹ and that of AN117 was 2.3 × 10⁹ M⁻¹.

### Example 4

### Specificity of Monoclonal Antibody

Using peptide and peptide fragment relating to α-hANP as a sample in α-hANP radioimmunoassay to be described in Example 5, cross reactivity was measured. The results are shown in Table 1. It was confirmed that both of AN111 and AN117 recognize the C-terminus of α-hANP.

**Table 1**

| Cross-Reactivity of ANP-Relating Polypeptide | | | |
|---|---|---|---|
| Polypeptide | Molecular Weight | Cross-reactivity (%) | |
| | | AN111 | AN117 |
| α-hANP | 3081 | 100 | 100 |
| β-hANP | 6162 | 142 | 136 |
| β′-hANP | 6162 | 112 | 108 |
| α-hANP[7-28] | 2393 | 61 | 60 |
| α-hANP[7-27] | 2231 | <0.2 | <0.2 |
| α-hANP[17-28] | 1302 | 115 | 113 |
| α-hANP[17-22] | 532 | <0.2 | <0.2 |
| α-hANP[17-20] | 343 | <0.2 | <0.2 |
| α-hANP[21-26] dimer | 1305 | <0.2 | <0.2 |
| α-hANP[23-28] dimer | 1578 | 228 | 221 |
| α-rANP | 3063 | 100 | 100 |
| α-rANP[7-23] | 1739 | <0.2 | <0.2 |

### Example 5

### Radioimmunoassay Using Monoclonal Antibody

### Materials

Assay buffer solution : 0.1 M phosphate buffer solution (pH 7.0) containing 0.372 g of disodium ethylenediamine tetraacetate, 0.063 g of cystine dihydrochloride, 0.262 g of ε-aminocaproic acid, 0.1g of sodium azide and 1 g of bovine serum albumin per liter.

Dilute ascitic fluid : AN111A 110,000-fold diluted with Assay buffer solution or AN117A 62,000-fold diluted with Assay buffer solution.

α-hANP standard : α-hANP serially two-fold diluted at a range of concentrations from 3.13 ng/ml to 100 ng/ml.

¹²⁵ I -labelled α-hANP : ¹²⁵I-labelled α-hANP (Amersham) diluted with Assay buffer solution up to approximately 3.7 KBq/ml.

1 % Bovine gamma globulin : dissolved in Assay buffer solution

37.5 % Polyethylene glycol : polyethylene glycol 6000 dissolved in Assay buffer solution without bovine serum albumin

### Methods

To a tube charged with 100 »ℓ of α-hANP standard or a sample, 100 »ℓ of ¹²⁵I-labelled α-hANP, 100 »ℓ of dilute ascitic fluid and 200 »ℓ of 1 % bovine gamma globulin were added and the mixture was incubated overnight at 4 °C. Immediately after 250 »ℓ of 37.5 % polyethylene glycol cooled to 4 °C is added thereto, the resulting mixture is agitated for 15 seconds and then centrifuged at 4 °C at 3000 rpm for 20 min. After removing the supernatant, the radioactivity of the precipitate is measured by a gamma counter (Aloka, ARC-600 type)

### Standard curve

Fig. 1 displays a standard curve of the radioimmunoassay. Sensitivities (90 % inhibition concentration) of AN111 and AN117 were 6.2 ng/ml and 4.4 ng/ml, respectively.

### Example 6

### Sandwich Radioimmunoassay of α-hANP

Anti-α-ANP [17-28] monoclonal antibody produced by the above hybridoma clone AN111 or AN117 (the antibody is hereinafter referred to as AN111 or AN117) which was immobilized on beads was used as an immobilized antibody. As a labelled antibody, anti-α-hANP [1 -28] monoclonal antibody produced by hybridoma clone KY-ANP-I (ECACC 87082001, EP-A-0 306 309) (the antibody is hereinafter referred to as KY-ANP-I) which was labelled with ¹²⁵I was used. By the above antibodies, a sandwich radioimmunoassay of α-hANP was established.

### 1. Purification of Ascitic Fluid

An ascitic fluid of AN111, AN117 or KY-ANP-I was purified by means of Affigel Protein A MAPS-II® (Bio-Rad). In brief, a mixture of 1 ml of an ascitic fluid and 1 ml of a binding buffer solution was loaded on Affigel Protein A® column (0.7 × 2 cm) which had previously been equilibrated with the same binding buffer solution. After washing the column with 30 ml of a binding buffer solution, 1 ml-fractions eluted were collected by the development with 100 ml of an eluting buffer solution. The fractions of which absorbance at 280 nm was above 0.1 were dialysed against distilled water to give an IgG fraction.

### 2. Preparation of Immobilized Antibody

In 0.05 M phosphate buffer (pH 7.4) containing the IgG fraction (100 »g/ml) of AN111A or AN117A prepared in Item 1 above are immersed polystyrene beads (Ichico, 3.2 mm diameter) and allowed to stand at 4 °C for 20 hr. After sufficiently washing the beads with RIA buffer solution (0.1 M phosphate buffer solution of pH 7.0 containing 0.1 % bovine serum albumin (crystal, Sigma, hereinafter referred to as BSA), 1 mM disodium ethylenediamine tetraacetate (hereinafter referred to as EDTA), 0.2 mM cystine and 0.1 % sodium azide), the beads were stored in the same buffer solution at 4 °C.

### 3. Preparation of ¹²⁵I-labelled antibody

With 10 »g of the IgG fraction of KY-ANP-I prepared in Item 1 above were mixed 50 »l of 0.5 M phosphate buffer solution (pH 7.5) and 0.5 mCi of Na¹²⁵I (Amersham). To the mixture was added 10 »l of 0.2 % chloramine T solution and agitated for 30 sec. After 10 »l of 1 % sodium metabisulfite was mixed therewith, 10 »l of 10 % potassium iodide and 10 »l of 1 % BSA solution was added to the mixture, followed by a gel-filtration (Sephadex G-25® (Fine, Pharmacia) : 0.9 × 25 cm, 0.1 M phosphate buffer solution of pH 7.4) to give 300 »Ci of ¹²⁵I-labelled antibody.

### 4. Assay

### a. Two-Step Assay

One piece of the antibody-immobilized beads in Item 2. 50 »l of α-hANP standard solution (10∼2000 pg/ml) or a human plasma sample and 100 »l of RIA buffer solution were mixed in a tube and incubated at 4 °C for 24 hr. After sucking the supernatant, the bead was washed 3 times with 1 ml of a washing solution (0.01 M phosphate buffer solution of pH 7.0 containing 0.1 % Tween 80 and 0.9 % sodium chloride). Then, 150 »l of ¹²⁵I-labelled antibody solution (1.2 × 10⁶ dpm/ml) was added thereto and incubated at 4 °C for 24 hr. After sucking the supernatant and washing the bead 3 times with 1 ml of the washing solution, radioactivity of ¹²⁵I-labelled antibody bound to the solid phase was measured by a gamma counter.

### b. One Step Assay

One piece of the antibody-immobilized beads prepared in Item 2 above was placed in a polystyrene tube, to which were added 50 »l of α-hANP standard solution (10 - 2,000 pg/ml) or a human plasma sample and 100 »l of ¹²⁵ I -labelled antibody solution (1.8 × 10⁶ dpm/ml) and incubated at 4 °C for 24 hr. After sucking the supernatant, the bead was washed 3 times with 1 ml of the washing solution. Radioactivity of ¹²⁵ I -labelled antibody bound to the solid phase was measured by a gamma counter.

A standard curve was prepared by plotting a concentration of α-hANP standard at abscissa and the measured radioactivity at ordinate. An ANP concentration in plasma was calculated by applying the radioactivity from the plasma sample to the standard curve.

The following results are from the one-step assay using AN111 as an immobilized antibody, unless otherwise defined.

### 5. Standard curve

Standard curve in the above assay 4-a and 4-b are shown in Figure 2. The minimum detection sensitivities of these assays were equally 0.5 pg/tube.

### 6. Cross-reactivity

Cross-reactivity to ANP-related compound is displayed in Figure 3 and Table 2. Cross-reactivity to ANP lacking the C-terminus such as α-hANP [7-27] and α-rANP was not observed.

### 7. Dilution Curve of Human Plasma

Two dilution curve of normal adults's plasma samples supplemented with α-hANP were parallel to the standard curve (Figure 4). Linear relationship focusing on the origin was obtained between plasma volume and measured quantity of ANP (Figure 5).

### 8. Recovery Test of α-hANP in Plasma

Average recovery of α-hANP added to two plasma samples of normal adults was 107.5 ± 7.6 % as shown in Table 3.

### 9. Concentration of α-hANP in Plasma

Average concentration of α-hANP in 5 plasma samples of normal adults was 9.6 ±6.5 pg/ml as displayed in Table 4.

**Table 2**

| Cross-Reactivity of ANP-Relating Peptide | |
|---|---|
| Peptide | Cross-Reactivity (Molar %) |
| α-hANP | 100 |
| β-hANP | 100 |
| β′-hANP | 100 |
| α-hANP[7-28] | 64 |
| α-hANP[7-27] | <0.01 |
| [Met(O)¹²]α-hANP | 27 |
| α-hANP[17-28] | <0.01 |
| α-hANP[17-28] dimer | <0.01 |
| α-rANP | 0.65 |

**Table 3**

| Recovery of α-hANP Added To Human Plasma | | | | |
|---|---|---|---|---|
| Added (pg/ml) | Plasma 1 | | Plasma 2 | |
| | Measured (pg/ml) | Recovery (%) | Measured (pg/ml) | Recovery (%) |
| 0 | 82.6 | ― | 18.1 | ― |
| 20 | 104 | 101 | 37.8 | 99 |
| 67 | 169 | 113 | 93.6 | 111 |
| 200 | 335 | 119 | 229 | 104 |
| 670 | 877 | 117 | 712 | 104 |
| 2000 | 2300 | 110 | 1970 | 97 |

**Table 4**

| α-hANP Concentration in Normal Adult Plasma by Two-Step Sandwich Radioimmunoassay | |
|---|---|
| Sample No. | pg/ml |
| 1 | <6.0 |
| 2 | 6.0 |
| 3 | 15.2 |
| 4 | 14.4 |
| 5 | 12.4 |
| Average | 9.6±6.5 |

### Example 7

### Sandwich Enzyme Immunoassay of α-hANP

The same immobilized antibody as in the above radioimmunoassay was used. As a labelled antibody, KY-ANP-I conjugated with peroxidase (from horseradish, Sigma, hereinafter referred to as HRP) according to the maleimide method (E. Ishikawa et al., J. Immunoassay, 4, 209-327 (1983)) was used. By using these antibodies, sandwich enzyme immunoassay of α-hANP was established.

### 1. Preparation of HRP-conjugated Antibody

### a. Preparation of KY-ANP-I Fab'

The IgG-fraction (5 mg) of KY-ANP-I as prepared above was dissolved in 0.1 M acetate buffer solution (pH 4.2) containing 0.1 M sodium chloride by addition of 0.2 mg of pepsin (from porcine gastric mucosa, Sigma) and incubated at 37 °C for 16 hr. The resulting mixture pH was adjusted to 7 with 2 M Tris-HCl buffer solution (pH 8.0) and then fractioned by gel-filtration (column : Ultrogel AcA 44® (LKB) 1.5 × 45 cm, elueut : 0.1 M phosphate buffer solution (pH 6.0) containing 5 mM EDTA) to give an F(ab')₂ fraction.

The F(ab')₂ fraction was concentrated up to 2 mg/0.4 ml by means of Centricon 30® (Amicon), to which was added 0.05 ml of 0.1 M 2-mercaptoethylamine solution. After incubation at 37 °C for 1.5 hr., the resulting mixture was subjected to gel-filtration (column; Sephadex G-25® (pharmacia), 0.9 × 45 cm, eluent; 0.1 M phosphate buffer solution (pH 6.0) containing 5 mM EDTA) for removing excess reagents. The resulting Fab' fraction was concentrated by means of Centricon 30®.

### b. Preparation of Maleimide-HRP

To 0.3 ml of 0.1 M phosphate buffer solution (pH 7.0) containing 2 mg of HRP was added 30 »l of dimethylformamide solution containing 10 mg/ml of N-succinimidyl 4-(N-maleimidomethyl)cyclohexanoate and agitated at 30 °C for an hour. After centrifugation (2000 × g, 5 min.) the supernatant was gel-filtrated in the same manner as Item 1-a (using Sephadex G-25®) for removing excess reagents. The resulting Maleimide-HRP fraction was concentrated to 1.8 mg/500 »l by means of Centricon 30®.

### c. Conjugation of HRP with KY-ANP-I Fab'

With 500 »l of 4 mg/ml KY-ANP-I Fab' solution in Item 1-a above was mixed with 500 »l of 3.6 mg/ml maleimide-HRP solution in Item 1-b above and incubated at 4 °C for 20 hr. After adding 0.1 M N-ethylmaleimide solution and incubating at 30 °C for an hour, the resulting mixture was gel-filtrated in the same manner as Item 1-a (using Ultrogel AcA 44®) to give HRP-conjugated antibody.

### 2. Assay

### a. Enzyme Immunoassay (Two-Step)

One piece of the antibody-immobilized beads was placed in a polystyrene tube, to which were added 0.05 ml of α-hANP standard solution (6 - 2000 pg/ml) or human plasma sample and 100 »l of EIA buffer solution (0.01 M phosphate buffer solution of pH 7.0, 0.1 % BSA, 1 mM EDTA, 0.2 mM cystine, 0.3 M sodium chloride and 1000 KIU/ml aprotinine (Sigma)), followed by incubation at 4 °C for 24 hr. After sucking the supernatant, the bead was washed twice with a washing solution (2 ml of 10 mM phosphate buffer solution of pH 7.0 containing 0.1 M sodium chloride), to which 150 »l of HRP-conjugated antibody solution 0.33 »g/ml was added followed by incubation at 4 °C for 24 hr. After sucking the supernatant the bead was washed twice with the 2 ml of a washing solution. Enzymatic activity of HRP-conjugated antibody bound to the solid phase was measured according to the following procedure b.

### b. Measurement of Enzymatic Activity

The bead was placed in another polystyrene tube, to which 100 »l of 0.1 M phosphate buffer solution (pH 8.0) containing 0.6 % 3-(4-hydroxyphenyl)propionate (Aldrich) and then 50 »l of 0.015 % hydrogen peroxide solution were added, followed by incubation at 30 °C for an hour. The reaction was terminated by addition of 2.5 ml of 0.1 M glycine buffer solution (pH 10.3). Fluorescence intensity was measured at excitation wavelength of 320 nm and fluorescence wavelength of 405 nm. The fluorescence intensity of 0.2 »g/ml quinine in 50 mM sulfuric acid solution was regarded as 100.

A standard curve was obtained by plotting concentrations of α-hANP standard solution at abscissa and fluorescence intensity at ordinate.

### 3. Standard Curve

Standard curves using AN111 and AN117 as immobilized antibodies are shown in Figure 6. Minimum detection sensitivities were equally 0.1 pg/tube.

## Claims (Claims for the following Contracting State(s): AT, BE, CH, DE, DK, FR, GB, GR, IT, LI, LU, NL, SE)

1. A monoclonal antibody recognizing the C-terminus of ANP obtainable from a hybridoma cell line which is
(a) AN111 having the deposit number FERM BP-2197; or
(b) AN117 having the deposit number FERM BP-2198.

2. The monoclonal antibody of claim 1 wherein said ANP is α-ANP or β-ANP.

3. The monoclonal antibody of claim 1 or 2 which is an antibody immobilized on a solid phase.

4. The monoclonal antibody of claim 3 in which said solid phase is a polystyrene bead.

5. Hybridoma AN111 (FERM BP-2197).

6. Hybridoma AN117 (FERM BP-2198).

7. An immunoassay for ANP which comprises sandwiching ANP between two antibodies, wherein one of said antibodies is the monoclonal antibody of any one of claims 1 to 4 and the other antibody recognizes the N-terminus of ANP.

8. The immunoassay of claim 7 which comprises using said monoclonal antibody immobilized on a solid phase for recognizing the C-terminus of ANP and the other antibody labelled with an enzyme or a radioisotope for recognizing the N-terminus of ANP.

9. The immunoassay of claim 7 or 8 wherein the antibody recognizing the N-terminus of ANP is a monoclonal antibody.

10. The immunoassay of claim 9 wherein said monoclonal antibody is KY-ANP-1 produced by hybridoma KY-ANP-1 (ECACC 87082001).

## Claims (Claims for the following Contracting State(s): ES)

1. A method for the preparation of a monoclonal antibody recognizing the C-terminus of ANP comprising immunization techniques, hybridoma techniques and purification techniques, wherein said antibody is obtainable from a hybridoma cell line which is
(a) AN111 having the deposit number FERM BP-2197; or
(b) AN117 having the deposit number FERM BP-2198.

2. The method according to claim 1, wherein said ANP is α-ANP or β-ANP.

3. The method of claim 1 or 2 wherein said monoclonal antibody is immobilized on a solid phase after purification.

4. The method of claim 3 in which said solid phase is a polystyrene bead.

5. Hybridoma AN111 (FERM BP-2197).

6. Hybridoma AN117 (FERM BP-2198).

7. An immunoassay for ANP which comprises sandwiching ANP between two antibodies, wherein one of said antibodies is the monoclonal antibody of any one of claims 1 to 4 and the other antibody recognizes the N-terminus of ANP.

8. The immunoassay of claim 7 which comprises using said monoclonal antibody immobilized on a solid phase for recognizing the C-terminus of ANP and the other antibody labelled with an enzyme or a radioisotope for recognizing the N-terminus of ANP.

9. The immunoassay of claim 7 or 8 wherein the antibody recognizing the N-terminus of ANP is a monoclonal antibody.

10. The immunoassay of claim 9 wherein said monoclonal antibody is KY-ANP-1 produced by hybridoma KY-ANP-1 (ECACC 87082001).

## Patentansprüche (Patentansprüche für folgende(n) Vertragsstaat(en): AT, BE, CH, DE, DK, FR, GB, GR, IT, LI, LU, NL, SE)

1. Monoclonaler Antikörper, der den C-Terminus von ANP erkennt, erhältlich aus einer Hybridom-Zellinie, die
(a) AN111 mit der Hinterlegungsnr. FERM BP-2197; oder
(b) AN117 mit der Hinterlegungsnr. FERM BP-2198 ist.

2. Monoclonaler Antikörper nach Anspruch 1, wobei das ANP α-ANP oder β-ANP ist.

3. Monoclonaler Antikörper nach Anspruch 1 oder 2, der ein auf einer festen Phase immobilisierter Antikörper ist.

4. Monoclonaler Antikörper nach Anspruch 3, wobei die feste Phase ein Polystyrol-Kügelchen ist.

5. Hybridom AN111 (FERM BP-2197).

6. Hybridom AN117 (FERM BP-2198).

7. Immuntest für ANP, umfassend das Bilden eines Sandwiches aus ANP zwischen zwei Antikörpern, wobei einer der Antikörper der monoclonale Antikörper nach einem der Ansprüche 1 bis 4 ist und der andere Antikörper den N-Terminus von ANP erkennt.

8. Immuntest nach Anspruch 7, umfassend die Verwendung des auf einer festen Phase immobilisierten monoclonalen Antikörpers zum Erkennen des C-Terminus von ANP und des mit einem Enzym oder einem Radioisotop markierten anderen Antikörpers zum Erkennen des N-Terminus von ANP.

9. Immuntest nach Anspruch 7 oder 8, wobei der den N-Terminus von ANP erkennende Antikörper ein monoclonaler Antikörper ist.

10. Immuntest nach Anspruch 9, wobei der monoclonale Antikörper der vom Hybridom KY-ANP-1 (ECACC 87082001) hergestellte monoclonale Antikörper KY-ANP-1 ist.

## Patentansprüche (Patentansprüche für folgende(n) Vertragsstaat(en): ES)

1. Verfahren zur Herstellung eines den C-Terminus von ANP erkennenden monoclonalen Antikörpers umfassend Immunisierungs-, Hybridom- und Reinigungstechniken, wobei der Antikörper aus einer Hybridom-Zellinie erhältlich ist, die
(a) AN111 mit der Hinterlegungsnr. FERM BP-2197; oder
(b) AN117 mit der Hinterlegungsnr. FERM BP-2198 ist.

2. Verfahren nach Anspruch 1, wobei das ANP α-ANP oder β-ANP ist.

3. Verfahren nach Anspruch 1 oder 2, wobei der monoclonale Antikörper nach der Reinigung auf einer festen Phase immobilisiert wird.

4. Verfahren nach Anspruch 3, wobei die feste Phase ein Polystyrol-Kügelchen ist.

5. Hybridom AN111 (FERM BP-2197).

6. Hybridom AN117 (FERM BP-2198).

7. Immuntest für ANP, umfassend das Bilden eines Sandwiches aus ANP zwischen zwei Antikörpern, wobei einer der Antikörper der monoclonale Antikörper nach einem der Ansprüche 1 bis 4 ist und der andere Antikörper den N-Terminus von ANP erkennt.

8. Immuntest nach Anspruch 7, umfassend die Verwendung des auf einer festen Phase immobilisierten monoclonalen Antikörpers zum Erkennen des C-Terminus von ANP und des mit einem Enzym oder einem Radioisotop markierten anderen Antikörpers zum Erkennen des N-Terminus von ANP.

9. Immuntest nach Anspruch 7 oder 8, wobei der den N-Terminus von ANP erkennende Antikörper ein monoclonaler Antikörper ist.

10. Immuntest nach Anspruch 9, wobei der monoclonale Antikörper der vom Hybridom KY-ANP-1 (ECACC 87082001) hergestellte monoclonale Antikörper KY-ANP-1 ist.

## Revendications (Revendications pour l'(les) Etat(s) contractant(s) suivant(s): AT, BE, CH, DE, DK, FR, GB, GR, IT, LI, LU, NL, SE)

1. Anticorps monoclonal reconnaissant l'extremité C-terminale de l'ANP susceptible d'être obtenu à partir d'une lignée de cellules d'hybridome qui est
(a) AN111 ayant le numéro de dépot FERM BP-2197; ou
(b) AN117 ayant le numéro de dépot FERM BP-2198.

2. Anticorps monoclonal selon la revendication 1, caractérisé en ce que l'ANP est l'ANP-α ou l'ANP-β.

3. Anticorps monoclonal selon la revendication 1 ou 2, caractérisé en ce qu'il s'agit d'un anticorps immobilisé sur une phase solide.

4. Anticorps monoclonal selon la revendication 3, caractérisé en ce que ladite phase solide est une bille de polystyrène.

5. Hybridome AN111 (FERM BP-2197).

6. Hybridome AN117 (FERM BP-2198).

7. Immunodosage pour l'ANP comprenant la mise en sandwich d'ANP entre deux anticorps, caractérisé en ce que l'un desdits anticorps est l'anticorps monoclonal selon l'une quelconque des revendications 1 à 4 et l'autre anticorps reconnaît l'extrémité N-terminale de l'ANP.

8. Immunodosage selon la revendication 7, caractérisé en ce qu'il comprend l'utilisation dudit anticorps monoclonal immobilisé sur une phase solide pour la reconnaissance de l'extrémité C-terminale de l'ANP et l'autre anticorps marqué par une enzyme ou un radioisotope pour la reconnaissance de l'extrémité N-terminale de l'ANP.

9. Immunodosage selon la revendication 7 ou 8, caractérisé en ce que l'anticorps reconnaissant l'extrémité N-terminale de l'ANP est un anticorps monoclonal.

10. Immunodosage selon la revendication 9, caractérisé en ce que ledit anticorps monoclonal est KY-ANP-1 produit par l'hybridome KY-ANP-1 (ECACC 87082001).

## Revendications (Revendications pour l'(les) Etat(s) contractant(s) suivant(s): ES)

1. Procédé de préparation d'un anticorps monoclonal reconnaissant l'extrémité C-terminale de l'ANP comprenant des techniques d'immunisation, des techniques d' hybridome et des techniques de purification, caractérisé en ce que ledit anticorps est susceptible d'être obtenu à partir d'une lignée de cellules d'hybridome qui est
(a) AN111 ayant le numéro de dépot FERM BP-2197; ou
(b) AN117 ayant le numéro de dépot FERM BP-2198.

2. Procédé selon la revendication 1, caractérisé en ce que l'ANP est l'ANP-α ou l'ANP-β.

3. Procédé selon la revendication 1 ou 2, caractérisé en ce que ledit anticorps monoclonal est immobilisé sur une phase solide après purification.

4. Procédé selon la revendication 3, caractérisé en ce que ladite phase solide est une bille de polystyrène.

5. Hybridome AN111 (FERM BP-2197).

6. Hybridome AN117 (FERM BP-2198).

7. Immunodosage pour l'ANP comprenant la mise en sandwich d'ANP entre deux anticorps, caractérise en ce que l'un desdits anticorps est l'anticorps monoclonal selon l'une quelconque des revendications 1 à 4 et l'autre anticorps reconnaît l'extrémité N-terminale de l'ANP.

8. Immunodosage selon la revendication 7, caractérisé en ce qu'il comprend l'utilisation dudit anticorps monoclonal immobilisé sur une phase solide pour la reconnaissance de l'extrémité C-terminale de l'ANP et l'autre anticorps marqué par une enzyme ou un radioisotope pour la reconnaissance de l'extrémité N-terminale de l'ANP.

9. Immunodosage selon la revendication 7 ou 8, caractérisé en ce que l'anticorps reconnaissant l'extrémité N-terminale de l'ANP est un anticorps monoclonal.

10. Immunodosage selon la revendication 9, caractérisé en ce que ledit anticorps monoclonal est KY-ANP-1 produit par l'hybridome KY-ANP-1 (ECACC 87082001).
